# EUROPÄISCHE PATENTSCHRIFT

(11) **EP 3 454 942 B1**
(45) Veröffentlichungstag und Bekanntmachung des Hinweises auf die Patenterteilung: **28.04.2021**
(21) Anmeldenummer: 17723329.3
(22) Anmeldetag: 09.05.2017
(51) Int. Cl.: A61N 2/02, A61N 2/00, G01R 33/34, G01R 33/44

(54) **VORRICHTUNG ZUR KERNSPINRESONANZTHERAPIE**
APPARATUS FOR NUCLEAR MAGNETIC RESONANCE THERAPY
DISPOSITIF DE THÉRAPIE PAR RÉSONANCE MAGNÉTIQUE NUCLÉAIRE

(30) Priorität: 10.05.2016 DE 102016108601
(43) Veröffentlichungstag der Anmeldung: 20.03.2019
(73) Patentinhaber: Muntermann, Axel, 35580 Wetzlar (DE)
(72) Erfinder: Muntermann, Axel, 35580 Wetzlar (DE)
(74) Vertreter: Blumbach · Zinngrebe Patentanwälte PartG mbB
(86) Internationale Anmeldenummer: PCT/EP2017/061037
(87) Internationale Veröffentlichungsnummer: WO 2017/194530

(56) Entgegenhaltungen:
- AT-B- 336 174
- DE-A1- 2 314 573
- DE-A1- 4 026 173
- DE-A1- 19 827 736
- DE-A1-102008 029 415
- DE-A1-102009 060 544
- DE-A1-102011 075 454
- US-A- 5 221 902
- US-A- 5 261 403
- US-A1- 2012 098 541

## Beschreibung

### Gebiet der Erfindung

Die Erfindung betrifft eine Vorrichtung zur Kernspinresonanztherapie, also eine Vorrichtung, mittels der sich Kernspinresonanzen zu Therapiezwecken im zu behandelnden Gewebe erzielen lassen.

### Hintergrund der Erfindung

Vorrichtungen zur Kernspinresonanztherapie sind bekannt. So zeigt beispielsweise die Deutsche Patentschrift DE 10 2009 060 544 B2 eine Vorrichtung, mittels der sich Kernspinresonanzen in einem zu behandelnden Gewebe zu Therapiezwecken erzielen lassen.

Die Druckschrift US 5 261 403 A beschreibt ein Verfahren zur Magnetresonanztomographie eines Körperteils oder -bereichs. Dieses Verfahren gehört damit, anders als die vorliegende Erfindung, zu den bildgebenden Verfahren, welche mit anderen Magnetfeldern arbeiten.

Die Druckschrift US 5 221 902 A beschäftigt sich ebenfalls mit der Magnetresonanztomographie und gehört damit ebenso zu den bildgebenden Verfahren.

Die Druckschrift DE 10 2011 075 454 A1 beschreibt ein weiteres bildgebendes MRT-Verfahren mit einer Lokalspule für ein Magnetresonanztomographiesystem für den Kopf- oder Halsbereich.

Die Druckschrift DE 198 27 736 A1 beschreibt eine Vorrichtung zur Behandlung von biologischem Material mit magnetischen Feldern.

Die Druckschrift DE 40 26 173 A1 beschreibt ein Verfahren für eine elektromagnetische Therapie zur Behandlung von Schlaganfällen, wobei eine Vorrichtung vorgesehen ist, mit welcher der Kopf eines Patienten behandelt werden kann.

Die Druckschrift US 2012/098541 A1 beschreibt eine Kompensationsspule zum Kompensieren des Magnetfelds eines Magnetresonanzsystems durch Generieren eines entsprechenden Magnetfelds. Zugrunde liegt dabei ein bildgebendes Verfahren.

Die in dieser Patentschrift gezeigte Vorrichtung hat den Vorteil, dass sie nach oben hin offen ist und sich der Benutzer nicht in eine Art Röhre legen muss, welche eine Ringspule aufnimmt.

Vorrichtungen zur Kernspinresonanztherapie arbeiten in der Regel mit im Vergleich zu bildgebenden NMR-Geräten niedrigen Feldstärken. Es wird im Bereich des zu behandelnden Gewebes ein möglichst homogenes Feld mit einer Feldstärke im mT-Bereich erzeugt. Dieses Feld setzt sich aus einem Sockelbetrag und einem zusätzlichen Feld zusammen, welches um einen gewissen Betrag gesweept wird, beispielsweise in Form eines Sägezahns. Dieses Feld bildet im Sinne der Theorie der Kernspinresonanzen das statische Feld. Senkrecht hierzu wird zumindest während der fallenden Flanken des gesweepten Feldes ein magnetisches Wechselfeld eingestrahlt, dessen Frequenz derart auf die Feldstärke des gesweepten Feldes abgestimmt ist, dass es beim Sweepen jeweils während des Abfalls des gesweepten Feldes zu einem Umklappen der Spins im zu behandelnden Gewebe kommt.

Es konnte wissenschaftlich belegt werden, dass über eine derartige Behandlung eine Regeneration von Gewebeschäden, insbesondere von Knorpel- und Knochenschäden, erzielt werden kann. Auch im kosmetischen Bereich zur Behandlung von Cellulite ist diese Therapieform verwendbar.

Besonders große Erfolge konnten im Bereich der Arthrosebehandlung erzielt werden. Es hat sich gezeigt, dass sich Knorpelgewebe aufgrund der Behandlung neu bilden kann, so dass Knorpelschäden regeneriert werden können.

Insbesondere bei der Behandlung von Arthrose ist es wünschenswert, ein möglichst großes Behandlungsvolumen bereitzustellen, da sich, insbesondere bei älteren Patienten, Knorpelschäden oft über viele Bereiche des Körpers erstrecken.

### Aufgabe der Erfindung

Vor diesem Hintergrund liegt der Erfindung die Aufgabe zugrunde, eine Vorrichtung zur Kernspinresonanztherapie bereitzustellen, welche ein möglichst großes Behandlungsvolumen bereitstellt und in welche der Benutzer möglichst komfortabel und bequem einsteigen kann.

Die Vorrichtung soll insbesondere für Arthrosepatienten mit Bewegungseinschränkungen gut geeignet sein.

### Zusammenfassung der Erfindung

Die Aufgabe der Erfindung wird bereits durch eine Vorrichtung zur Kernspinresonanztherapie nach Anspruch 1 gelöst.

Bevorzugte Ausführungsformen und Weiterbildungen der Erfindung sind dem Gegenstand der abhängigen Ansprüche zu entnehmen.

Die Erfindung betrifft eine Vorrichtung zur Kernspinresonanztherapie, also eine vom Grundprinzip bekannte und eingangs beschriebene Vorrichtung, mittels der sich Kernspinresonanzen im zu behandelnden Gewebe erzielen lassen, indem ein Magnetfeld, welches sich möglichst homogen durch ein Behandlungsvolumen erstreckt, gesweept wird und gleichzeitig senkrecht hierzu ein magnetisches Wechselfeld eingestrahlt wird, zumindest während das gesweepte Feld fällt.

Die Feldstärke des gesweepten Feldes beträgt dabei insbesondere zwischen 0,1 bis 100 mT, bevorzugt 0,3 und 3 mT. Dies geht mit einer Frequenz des Wechselfeldes von 1 bis 100 kHz, bevorzugt 10 bis 100 kHz, einher.

Die maximale Feldstärke des Wechselfeldes liegt vorzugsweise zwischen 0,1 bis 100 mT, bevorzugt zwischen 0,1 und 3 mT.

Zur Aufnahme eines Patienten umfasst die Vorrichtung zur Kernspinresonanztherapie eine Liege. Diese ist vorzugsweise im Wesentlichen horizontal angeordnet. Es ist aber auch denkbar, dass es sich um eine Art Liegesessel handelt, bei welchem insbesondere die Liege ein Oberteil aufweist, welches im steileren Winkel als das Unterteil steht.

Seitlich neben der Liege ist jeweils eine Spule, insbesondere eine Sweepspule angeordnet, wobei durch den Raum zwischen den Spulen ein Behandlungsvolumen erzielt wird.

Diese Spulen stehen vorzugsweise im Wesentlichen in Helmholtz-Konfiguration und erzeugen so ein im Wesentlichen homogenes Feld, welches quer über die Liege verläuft.

Als Sweepspulen werden vorzugsweise Kupferspulen mit jeweils 10 bis 300 Windungen verwendet.

Vorzugsweise wird das Sweepfeld, welches, wie eingangs beschrieben, aus einem Sockelbetrag und einem modulierten Betrag besteht, allein durch die Sweepspulen erzeugt.

Es ist aber auch denkbar, dass der Sockelbetrag des Sweepfeldes ganz oder teilweise durch Permanentmagnete erzeugt wird, welche vorzugsweise ebenfalls in den Seitenteilen der Vorrichtung zur Kernspinresonanztherapie angeordnet sind.

Um innerhalb des Behandlungsvolumens, das zwischen den Sweepspulen vorhanden ist, Kernspinresonanzen zu erzeugen, wird senkrecht zu dem Sweepfeld ein magnetisches Wechselfeld erzeugt.

Hierzu umfasst die erfindungsgemäße Vorrichtung zur Kernspinresonanztherapie eine erste Spule zur Erzeugung eines magnetischen Felder, insbesondere eines Wechselfeldes, welche unterhalb der Liege angeordnet ist.

Eine zweite Spule zur Erzeugung eines magnetischen Feldes, insbesondere eines Wechselfeldes, ist über dem Behandlungsvolumen angeordnet.

Diese zweite Spule befindet sich also vorzugsweise oberhalb oder direkt angrenzend an die Sweepspulen.

Die erste und die zweite weitere Spule zur Erzeugung eines magnetischen Wechselfeldes sind vorzugsweise ebenfalls in Helmholtz-Konfiguration angeordnet. Diese befinden sich im Behandlungsfall oberhalb und unterhalb des Patienten. Hierdurch wird ein magnetisches Wechselfeld im Behandlungsvolumen erzeugt, das über das gesamte Behandlungsvolumen gegenüber einer einzelnen Spule zur Erzeugung eines magnetischen Wechselfeldes eine höhere Feldstärke innerhalb des gesamten Behandlungsvolumens aufweist.

Die Erfindung betrifft also eine Vorrichtung zur Behandlung mit magnetischen Feldern, mittels welcher Kernspinresonanzen im zu behandelnden Gewebe erzeugbar sind, wobei vorzugsweise mittels seitlich angeordneter Spulen ein im Wesentlichen statisches Feld erzeugt wird und hierzu senkrecht ein Wechselfeld mit zwei Spulen erzeugt wird, von welchen sich eine unterhalb und eine oberhalb des Benutzers befindet.

Im Sinne der Erfindung ist aber auch genau eine umgekehrte Anordnung denkbar, bei welcher sich die Spulen zur Erzeugung des Wechselfeldes seitlich und die Spulen zur Erzeugung des im Wesentlichen statischen Feldes oberhalb und unterhalb des Benutzers befinden.

Die Spulen zur Erzeugung des magnetischen Wechselfeldes sind vorzugsweise im Wesentlichen genauso lang wie die Sweepspulen. Insbesondere haben sie eine Länge der Sweepspulen +/- 20 %.

Bei einer anderen Ausführungsform der Erfindung ist die zweite Spule zur Erzeugung des magnetischen Wechselfeldes hochklapp- oder hochfahrbar ausgebildet.

Insbesondere ist die obere Spule zur Erzeugung eines magnetischen Wechselfeldes an dem Gehäuse einer Sweepspule angeschlagen und um einen Winkel von mindestens 30°, vorzugsweise mindestens 40°, nach oben klappbar. Insbesondere Patienten mit Bewegungseinschränkungen können die Liege so leichter erreichen.

Die obere bzw. zweite Spule zur Erzeugung eines magnetischen Wechselfeldes ist bei einer Ausführungsform der Erfindung in einem ringförmigen, innen offenen Gehäuse mit vorzugsweise in einer Draufsicht im Wesentlichen rechteckigen Form angeordnet.

Es handelt sich mithin um eine Rat Rahmen, in welchem die obere Spule zur Erzeugung des Wechselfeldes angeordnet ist.

Dieser Rahmen hat vorzugsweise einen Durchmesser von weniger als 200, besonders bevorzugt weniger als 100 mm. So ist innerhalb des Rahmens eine Fläche frei, wodurch die Vorrichtung zur Kernspinresonanztherapie trotz der oberen Spule nach oben hin größtenteils offen ist. Patienten, die in der erfindungsgemäßen Vorrichtung liegen, fühlen sich so nicht eingeengt.

Eine obere Spule über dem Behandlungsvolumen folgt vorzugsweise im Wesentlichen der Form der Liege und erstreckt sich insbesondere über eine Länge von 70 % der Liegefläche.

Die Spulen zur Erzeugung des magnetischen Wechselfeldes und/oder zur Erzeugung des Sweepfeldes können insbesondere in einem Gehäuse angeordnet sein, welches in seiner Haupterstreckungsrichtung zueinander parallellaufende Seiten hat, in welchen die Spulen geradlinig verlaufen, wobei an den Schmalseiten die Spulen bogenförmig, insbesondere in Form eines Kreisbogens verlaufen. Das Gehäuse ist vorzugsweise in der Mitte zumindest teilweise offen.

So lässt sich ein großes, an das über einer Liege angeordnete Behandlungsvolumen angepasstes Magnetfeld auf einfache Weise erzeugen.

Bei einer bevorzugten Ausführungsform der Erfindung sind zwei Spulen, insbesondere eine Sweepspule und die zweite weitere Spule, welche vorzugsweise der Erzeugung eines magnetischen Wechselfeldes dient, an einem hochklappbaren Winkelteil angebracht.

Es ist mithin gemäß dieser Ausführungsform der Erfindung vorgesehen, dass zwei der insgesamt 4 Spulen, die der Erzeugung von Kernspinresonanzen im Behandlungsvolumen dienen, zusammen hochgeklappt werden, so dass der Benutzer die vorzugsweise als Liege ausgebildete Vorrichtung leicht betreten kann.

Es ist insbesondere vorgesehen, dass das Winkelteil über ein Drehgelenk mit der übrigen Vorrichtung verbunden ist.

Um einen großen Einstiegsbereich bereitzustellen, ist gemäß einer bevorzugten Ausführungsform der Erfindung das Winkelteil um einen Winkel von mindestens 50°, vorzugsweise von mindestens 70°, nach oben schwenkbar ausgebildet.

Das Winkelteil kann in einer nach oben geklappten Position mittels Federn, insbesondere mittels Gaszugfedern, in Position gehalten werden. Über derartige Zugfedern kann ein leichtes Hoch- und Herunterklappen sichergestellt werden.

Vorzugsweise umfasst die Vorrichtung zumindest zwei, besonders bevorzugt vier Zugfedern. Hierdurch kann beispielsweise bei einem Versagen einer Zugfeder sichergestellt werden, dass das Winkelteil mit den beiden Spulen nicht mit seinem gesamten Gewicht nach unten fällt. Bei einer Weiterbildung der Erfindung umfasst das Winkelteil zumindest ein Bedienteil, insbesondere einen Bildschirm, welcher beispielsweise als Touchscreen ausgebildet sein kann.

Das Bedienteil kann insbesondere an einer oberen Ecke des Winkelteils angeordnet sein und ist gemäß einer bevorzugten Ausführungsform sowohl gegenüber der horizontalen als auch gegenüber der vertikalen Ebene schräg gestellt, so dass das Winkelteil schräg dem Bediener der Vorrichtung zugewandt ist.

Insbesondere kann die Oberfläche eines Bildschirms gegenüber einer vertikalen Ebene um einen Winkel von 20-80°, bevorzugt von 30-70°, verkippt sein.

Im Winkelteil können noch weitere elektronische Steuerungskomponenten, insbesondere ein Steuergerät zur Ansteuerung der Spulen, angeordnet sein. Vorzugsweise befinden sich schwere Komponenten des Steuergeräts nicht im beweglichen Winkelteil, sondern sind unterhalb der Liegefläche der Vorrichtung angeordnet.

Bei einer Weiterbildung der Erfindung ist zumindest eine Spule in einer Vergussmasse, insbesondere in einem Harz, eingebettet.

Es ist insbesondere vorgesehen, dass die Spulen in einem vorzugsweise aus Kunststoff ausgebildeten Gehäuse eingelegt und mit einer Vergussmasse vergossen sind.

Durch die Verwendung einer Vergussmasse wird eine Bewegung der Spulen, insbesondere aufgrund von Lorentzkräften, vermieden. So können insbesondere störende Brummgeräusche eliminiert werden.

Als Kunststoff für das Gehäuse wird vorzugsweise ein faserverstärktes Verbundmaterial, insbesondere GFK oder CFK, verwendet.

Bei einer Weiterbildung der Erfindung umfasst die Vorrichtung eine austauschbare Liegefläche. Die Liegefläche kann so gegen eine andere Liegefläche mit einer anderen Form oder mit weiteren funktionellen Behandlungskomponenten, z.B. Gurte zur Fixierung eines Körperteils, getauscht werden.

Bei einer Weiterbildung der Erfindung umfasst die Vorrichtung eine Beleuchtungseinrichtung mit veränderbarer Farbe. Diese ist vorzugsweise als LED-Leuchtstreifen ausgebildet.

Es ist insbesondere vorgesehen, dass sich im Betrieb der Vorrichtung, also beim Erzeugen von Kernspinresonanzen, die Lichtfarbe ändert.

Weiter kann die Beleuchtungseinrichtung der Markierung des Behandlungsvolumens dienen, z.B. indem zwei Leuchtstreifen, die an der zweiten weiteren Spule angeordnet sind, seitliche Lichtstreifen auf die Liegefläche projizieren.

Bei einer Weiterbildung der Erfindung umfasst die Vorrichtung ein Steuergerät, mit welchem zumindest bei einem Start der Vorrichtung die Stromstärke einer der Spulen und/oder die Frequenz eines von zumindest einer der Spulen erzeugten Wechselfeldes gemessen wird.

Diese Ausführungsform der Erfindung macht sich hinsichtlich des Sweepfeldes die Tatsache zunutze, dass die Feldstärke des Sweepfeldes von der Stromstärke abhängig ist.

Über eine Strommessung kann so kontrolliert werden, ob die Feldstärke des Sweepfeldes im Sollbereich liegt.

Ist dies aufgrund eines Fehlers nicht der Fall, wäre das Erreichen einer Resonanzbedingung im Behandlungsvolumen nicht gewährleistet und das Gerät gibt eine Fehlermeldung aus und bricht die Behandlung ab.

Vorzugsweise wird auch das magnetische Wechselfeld hinsichtlich seiner Frequenz kontrolliert.

Die Kontrolle der Frequenz des Wechselfeldes und/oder die Kontrolle der Stromstärke erfolgt gemäß einer bevorzugten Ausführungsform der Erfindung auch während des laufenden Betriebs.

Bei einer bevorzugten Ausführungsform der Erfindung erfolgt die Überprüfung der Stromstärke und/oder die Überprüfung der Frequenz des Wechselfeldes für jede Spule einzeln. So kann bei einem etwaigen Fehler die Fehlerursache leicht eingegrenzt werden.

Bei einer Weiterbildung der Erfindung umfasst die Vorrichtung einen Sensor, insbesondere ausgebildet als Schalter, mittels welchem kontrolliert wird, ob die Vorrichtung geschlossen ist, also insbesondere, ob das Winkelteil zugeklappt ist.

Eine Behandlung kann so nur durchgeführt werden, wenn die Spulen in der richtigen Position zueinander sind und nicht etwa, wenn die Vorrichtung teilweise geöffnet ist.

Bei einer Ausführungsform der Erfindung umfasst das Steuergerät eine Zeitmesseinrichtung, die bei Unterbrechung einer Behandlung die Zeit stoppt und die Behandlung bis zu einer eingegebenen oder gespeicherten Gesamtbehandlungsdauer fortsetzt, wenn die Vorrichtung wieder in Betrieb genommen wird. So ist sichergestellt, dass auch bei Behandlungsunterbrechungen die gewünschte Behandlungszeit genau erreicht wird.

Bei einer Ausführungsform der Erfindung sind die Sweepspulen jeweils in einem Seitenteil, welches an die Liege angrenzt, angeordnet, wobei eines der Seitenteile runterfahr- oder hochklappbar ausgebildet ist.

Bei einer ersten Ausführungsform der Erfindung ist ein Seitenteil, in welchem sich eine Sweepspule befindet, nach unten fahrbar, so dass es im heruntergefahrenen Zustand vorzugsweise nicht wesentlich über die Liegefläche hinausragt. Nach Herunterfahren des Seitenteils und ggf. Hochklappen des Gehäuses der oberen Spule zur Erzeugung des Wechselfeldes kann der Patient bzw. Benutzer bequem in die Vorrichtung zur Kernspinresonanztherapie einsteigen. Vorzugsweise ist nur ein Seitenteil herunterfahr- oder hochklappbar ausgebildet. So wird die Mechanik lediglich in einem Seitenteil benötigt und mit dem feststehenden Seitenteil kann die Vorrichtung beispielsweise an eine Wand gestellt werden.

Bei einer Ausführungsform der Erfindung ist ein unterer Abschnitt der Sweepspulen gerade und ein oberer Abschnitt der Sweepspulen gebogen ausgebildet. Insbesondere weisen die Sweepspulen im Wesentlichen die Form eines Segmentbogenfensters auf. Die Kontur der Sweepspulen setzt sich mithin aus einem Rechteck und einem Kreisbogen oder Ellipsensegment zusammen.

Diese Form nähert sich der Anatomie des Benutzers an. In den hinteren und vorderen Bereichen, also in denjenigen Bereichen, in denen bei der Behandlung die Extremitäten angeordnet sind, wird ein weniger großes Behandlungsvolumen benötigt, als in einem mittleren Bereich.

Der Segmentbogen hat insbesondere einen Radius zwischen 1 und 2 m.

Bei einer Ausführungsform der Erfindung ist die Liege Teil eines abtrennbaren und um 180° drehbaren Moduls, welches zumindest die erste Spule zur Erzeugung eines magnetischen Wechselfeldes umfasst. Insbesondere umfasst das Modul mit der Liege auch die Steuereinrichtung zur Ansteuerung der Spulen sowie eine Bedieneinrichtung. Die Bedieneinrichtung ist vorzugsweise an einer Vorderseite oder Rückseite angeordnet.

Das Modul mit der Liege und der Steuereinrichtung kann mit den weiteren Spulen, also mit den Sweepspulen, sowie der oberen zweiten Spule zur Erzeugung eines magnetischen Wechselfeldes verbunden werden.

Je nach dem, wo die erfindungsgemäße Vorrichtung aufgestellt wird, kann das Modul mit der Liege um 180° gedreht eingebaut werden, so dass insbesondere das Bedienfeld oder eine Kopfstütze der Liege auf der gewünschten Seite angeordnet ist.

Die Seitenteile sind bei einer Weiterbildung der Erfindung als Gehäuse ausgebildet, welches jeweils eine Sweepspule aufnimmt. Zumindest bei einer herunterfahrbaren Sweepspule auf einer Seite befindet sich ein Sweepspulengehäuse vorzugsweise zwischen zwei Säulen, zwischen denen das Sweepspulengehäuse heruntergefahren wird.

Das durch die Vorrichtung zur Kernspinresonanztherapie bereitgestellte Behandlungsvolumen hat bei einer bevorzugten Ausführungsform der Erfindung eine Länge zwischen 1,4 und 2,5 m, eine Breite zwischen 0,4 und 1 m und/oder eine Höhe zwischen 0,4 und 1 m.

### Kurzbeschreibung der Zeichnungen

Der Gegenstand der Erfindung soll im Folgenden bezugnehmend auf die Zeichnungen Fig. 1 bis Fig. 15 näher erläutert werden.
Fig. 1 bis Fig. 8 zeigt eine erste Ausführungsform der Erfindung, bei welcher zwei Spulen in einem hochklappbaren Winkelteil angeordnet sind.
Fig. 1 und Fig. 2 sind perspektivische Ansichten einer Vorrichtung zur Kernspinresonanztherapie.
Fig. 3 ist eine Drahtdarstellung in Detailansicht.
Fig. 4 zeigt die Vorrichtung ohne Gehäuse.
Fig. 5 ist eine Detaildarstellung der Fig. 4.
Fig. 6 ist eine Detaildarstellung des Drehgelenks der Vorrichtung.
Fig. 7 ist eine perspektivische Darstellung, in welcher die Vorrichtung in geöffnetem Zustand dargestellt ist.

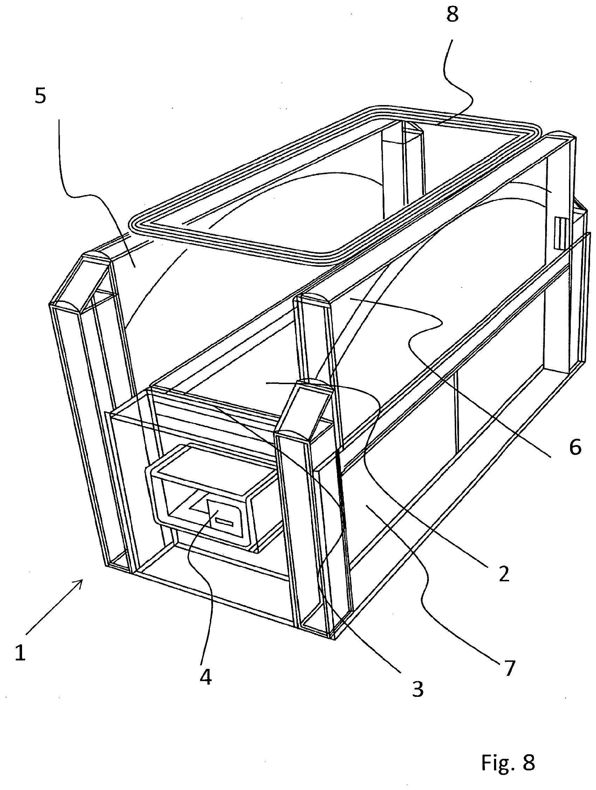

Bezugnehmend auf Fig. 8 bis Fig. 16 soll eine weitere Ausführungsform der Erfindung erläutert werden, bei welcher ein Seitenteil herunterfahrbar ausgebildet ist.

Fig. 8 zeigt eine perspektivische Ansicht einer Vorrichtung zur Kernspinresonanztherapie.

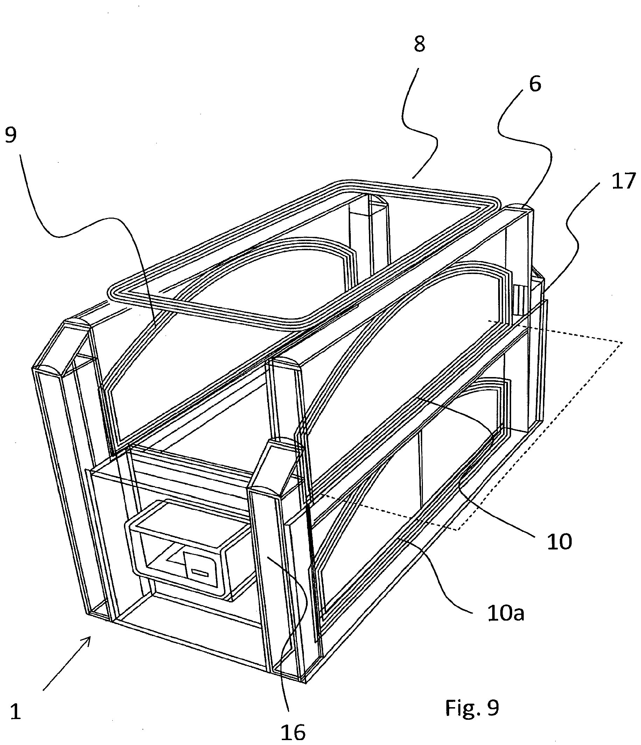

Fig. 9 zeigt eine weitere perspektivische Ansicht einer Vorrichtung zur Kernspinresonanztherapie in einer Drahtdarstellung, in welcher auch verdeckte Kanten eingeblendet sind.

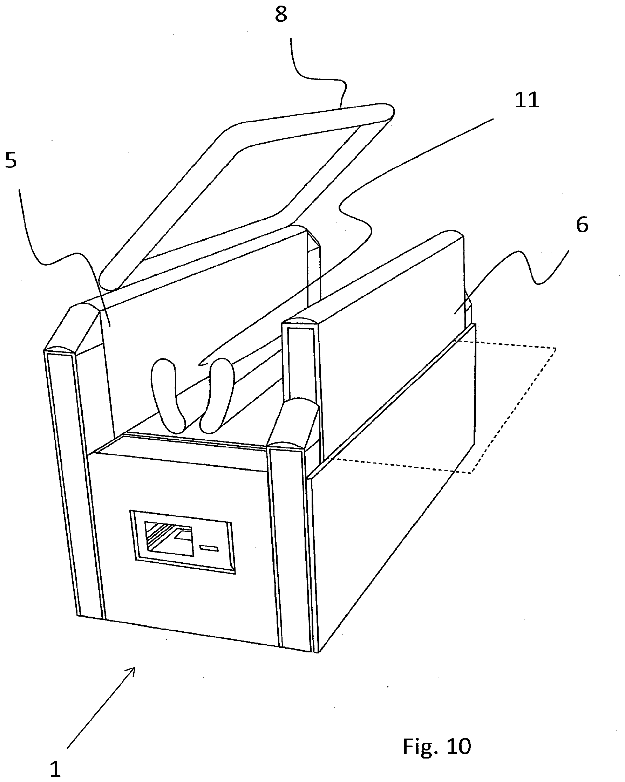

Fig. 10 zeigt die in Fig. 9 dargestellte Vorrichtung zur Kernspinresonanztherapie, wobei die obere Spule zur Erzeugung eines magnetischen Wechselfeldes nach oben geklappt ist.

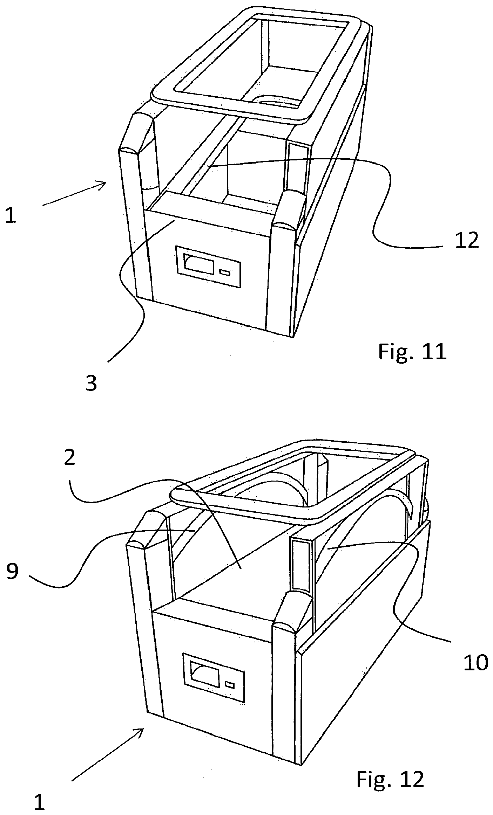

Fig. 11 und Fig. 12 zeigen zwei weitere perspektivische Ansichten einer Vorrichtung zur Kernspinresonanztherapie.

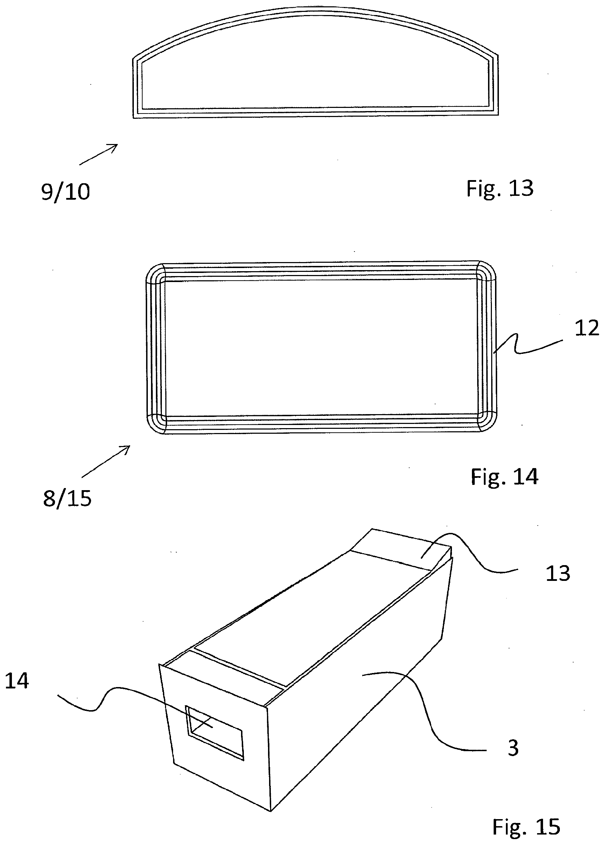

Fig. 13 ist die Darstellung einer Sweepspule.

Fig. 14 zeigt das Spulengehäuse einer Spule zur Erzeugung des magnetischen Wechselfeldes.

Fig. 15 zeigt eine perspektivische Ansicht eines Moduls, welches die Liege umfasst.

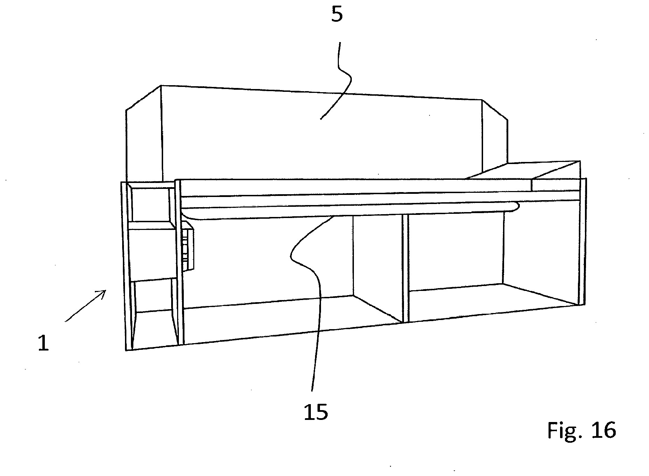

Fig. 16 zeigt eine Schnittansicht der Vorrichtung zur Kernspinresonanztherapie.

### Detaillierte Beschreibung der Zeichnungen

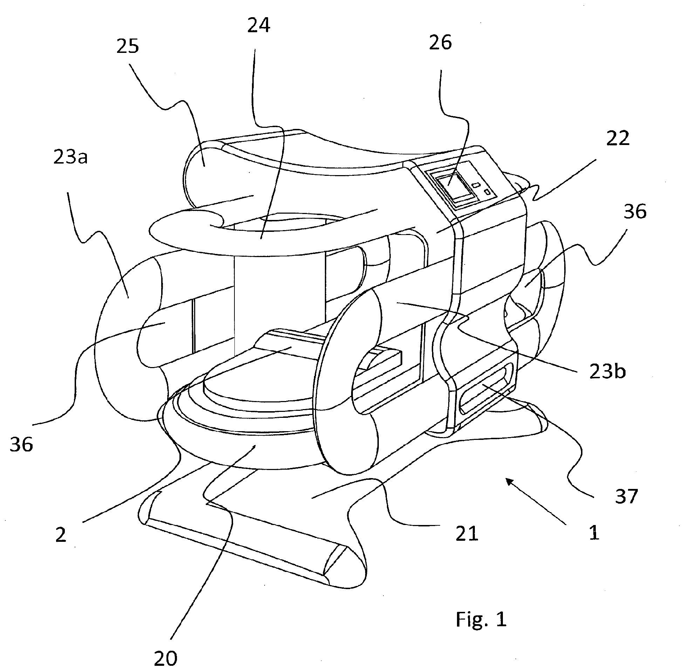

Fig. 1 ist eine perspektivische Ansicht einer ersten Ausführungsform einer Vorrichtung zur Kernspinresonanztherapie 1.

Die Vorrichtung zur Kernspinresonanztherapie 1 umfasst in diesem Ausführungsbeispiel eine Liege 20 mit einer Liegefläche 2, auf welche sich der Benutzer legen kann.

Die Liege 20 befindet sich auf einem Fußteil 21.

An die Seite der Liege 20 grenzen Spulengehäuse 23a, 23b an, welche in diesem Ausführungsbeispiel Spulen umfassen, die der Erzeugung des Sweepfeldes dienen.

Die Spulengehäuse 23a, 23b umfassen ein im Wesentlichen rechteckiges Mittelstück, bei welchem die Kanten der Gehäuse 23b, 23b parallel zueinander verlaufen. An den Schmalseiten sind die Gehäuse 23a, 23b gebogen, in diesem Ausführungsbeispiel in Form eines Kreisbogens ausgestaltet.

So ist es möglich, Spulengehäuse 23a und 23b bereitzustellen, welche im Wesentlichen dieselbe Länge wie die Liegefläche 2 haben und mit denen sich ein großes Behandlungsvolumen bereitstellen lässt.

Das Behandlungsvolumen wird durch das Volumen zwischen den Spulen 23a und 23b sowie durch den Bereich zwischen der Liegefläche 2 bis zum Spulengehäuse 24 definiert.

In der Liege 20 befindet sich eine Spule zur Erzeugung eines magnetischen Wechselfeldes.

Eine weitere Spule zur Erzeugung eines magnetischen Wechselfeldes befindet sich in dem Spulengehäuse 24, welches oberhalb der Spulengehäuse 23a und 23b angeordnet ist.

Das Spulengehäuse 24 ist ebenfalls langgestreckt mit abgerundeten Schmalseiten ausgebildet.

Auch die Liege 20 hat abgerundete Schmalseiten und es ist in der Liege eine Spule (hier nicht dargestellt) angeordnet, deren Form im Wesentlichen der Spule im Spulengehäuse entspricht.

Über die Spule im Spulengehäuse 24 wird ebenfalls ein magnetisches Wechselfeld erzeugt.

Die Spule in der Liege 20 sowie die Spule im Spulengehäuse 24 sind in Helmholtz-Konfiguration angeordnet und es wird innerhalb des Behandlungsvolumens ein magnetisches Wechselfeld mit homogener Feldstärke erzeugt.

So ist auf einfache Weise eine Ganzkörperbehandlung eines Benutzers möglich.

Sämtliche Spulengehäuse 23a, 23b, 24 umfassen eine Aussparung 36, die insbesondere dazu dient, einer Platzangst des Benutzers während der Behandlung entgegenzutreten.

Um in die Vorrichtung einsteigen zu können, umfasst die Vorrichtung 1 ein in diesem Ausführungsbeispiel im Wesentlichen mittig angeordnetes Winkelteil 22, welches über das als Scharnier ausgebildete Drehgelenk 25 nach oben geklappt werden kann.

An dem Winkelteil 22 angebracht ist das Spulengehäuse 24 der oberen Spule über dem Behandlungsvolumen sowie an seiner Frontseite das Spulengehäuse 23b der einen Sweepspule.

Beim Hochklappen des Winkelteils 22 wird sowohl das Spulengehäuse 23b als auch das Spulengehäuse 24 mit nach oben geklappt und der Benutzer kann leicht die Liegefläche 2 erreichen.

Unten am Winkelteil 22 befindet sich ein Griff 37, um die Vorrichtung 1 leicht öffnen und schließen zu können.

Das Winkelteil 22 erstreckt sich nur über einen Teilbereich der Aussparung 36.

An einer oberen Ecke des Winkelteils 22 befindet sich ein Bildschirm 26, welcher vorzugsweise als Touchscreen ausgebildet ist. Auch das Steuergerät (nicht dargestellt) kann sich im Winkelteil befinden.

Der Bildschirm bzw. die Oberfläche des Bildschirms 26 ist gegenüber einer vertikalen Ebene um einen Winkel vom 30-70° verkippt, um den Bildschirm, welcher vorzugsweise als Touchscreen ausgebildet ist, leicht erreichen zu können.

Auch weitere Steuerungskomponenten, wie beispielsweise ein Kartenlesegerät, können in diesem Bereich angeordnet sein.

Die Liegefläche 2 kann abgenommen und gegen eine andere Liegefläche (nicht dargestellt) getauscht werden, welche weitere Behandlungskomponenten umfasst.

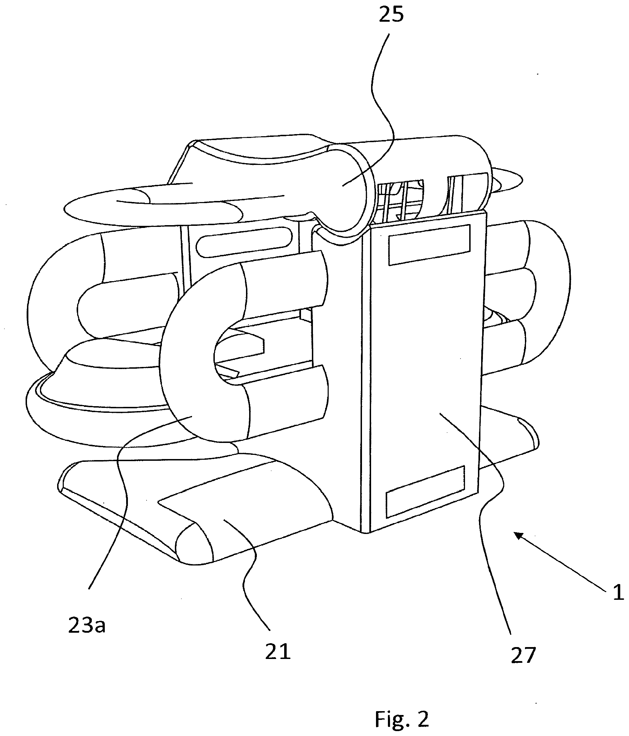

Fig. 2 ist eine perspektivische Ansicht der Rückseite der in Fig. 1 dargestellten Vorrichtung 1.

Zu erkennen ist in dieser Ansicht insbesondere, dass die Vorrichtung 1 ein mit dem Fußteil 21 verbundenes Rückenteil 27 aufweist, an welchem zum einen das Spulengehäuse 23a und zum anderen das Drehgelenk 25 angebracht ist.

Das Rückenteil 27 erstreckt sich in diesem Ausführungsbeispiel in etwa säulenartig mittig nach oben.

Es versteht sich, dass auch im Rückenteil 27 Steuerungskomponenten der Vorrichtung 1 angeordnet sein können.

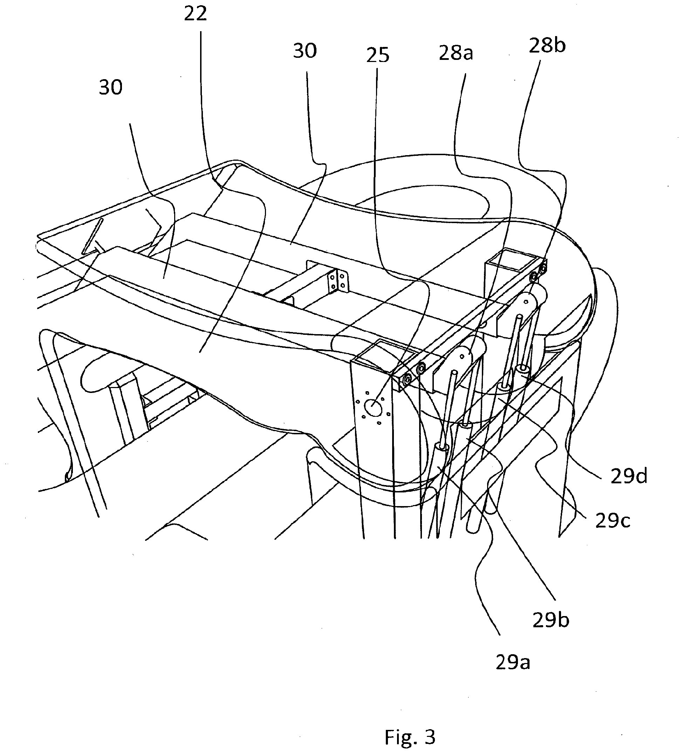

Fig. 3 ist eine perspektivische Drahtdarstellung, in welcher insbesondere das Drehgelenk 25 zu erkennen ist.

Das Winkelteil 22 besteht aus einem Gehäuse, in welchem Träger 30, vorzugsweise Träger 30 aus Metall, eingelassen sind.

Diese Träger 30 sind an dem Drehgelenk 25 drehbar gelagert.

Auf der dem Träger 30 gegenüberliegenden Seite der Drehachse befindet sich jeweils ein Arm 28a, 28b.

Die Arme 28a und 28b sind mit jeweils zwei Gaszugfedern 29a, 29b sowie 29c und 29d verbunden.

Über die Gaszugfedern 29a - 29d kann das Winkelteil 22 leicht nach oben geschwenkt werden. Durch das damit verbundene Herunterklappen der Arme 28a und 28b verlängert sich der Hebelarm, so dass im vollständig geöffneten Zustand von den Gaszugfedern 29a - 29c eine höhere Kraft als im geschlossenen Zustand der Vorrichtung ausgeht und die Vorrichtung selbständig geöffnet bleibt.

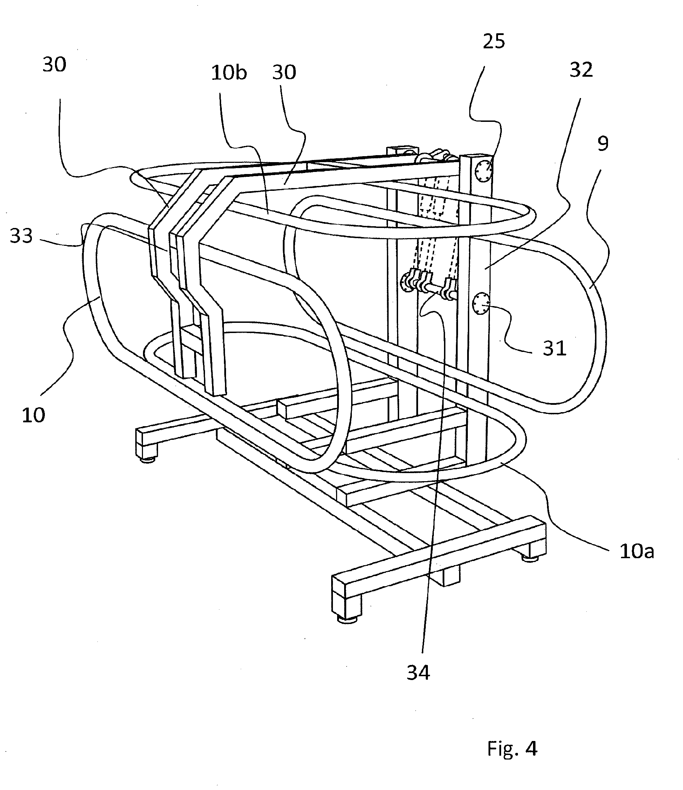

Fig. 4 zeigt eine perspektivische Ansicht, in welcher sämtliche Gehäusekomponenten und Teile der Liege ausgeblendet sind.

Zu erkennen sind die Spulen 9 und 10, die in Helmholtz-Konfiguration stehen und der Erzeugung des Sweepfeldes dienen.

Unterhalb der Liege befindet sich die Spule 10a und oberhalb der Liege die Spule 10b, über die ein senkrecht zum Sweepfeld verlaufendes Wechselfeld eingestrahlt wird.

Zu erkennen sind die Träger 30, welche winkelförmig ausgebildet und an dem Drehgelenk 25 angebracht sind.

Unterhalb des Drehgelenks 25 befindet sich eine in einem weiteren Drehgelenk 31 gelagerte Stange 34, an welcher das gegenüberliegende Ende der Gaszugfedern angebracht ist.

Der Rahmen 32 umfasst Träger sowohl für das Rückenteil als auch für die Liege.

Die schwenkbar an dem Rahmen 32 angeschlagenen und miteinander verbundenen Träger 30 umfassen an der Vorderseite einen Vorsprung 33, innerhalb dessen ein oberer Abschnitt der Spule 10 verläuft.

Die Metallteile des Rahmens 32 sowie der Träger 30 können, wie es bei einer Ausführungsform der Erfindung vorgesehen ist, elektrisch voneinander isoliert sein, um eine Induktion von Strömen gering zu halten.

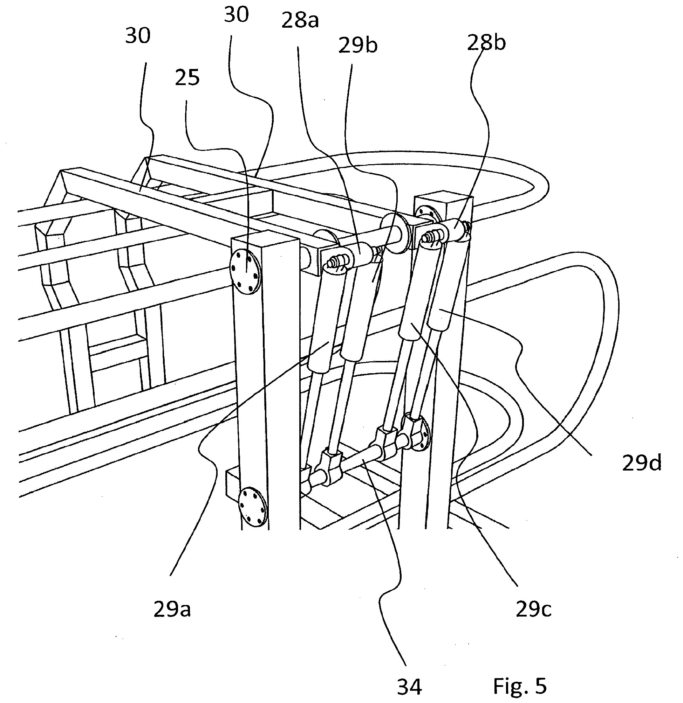

Fig. 5 ist eine Detaildarstellung der Fig. 4, in welcher nunmehr, von der Rückseite her gesehen, der Bereich des Drehgelenks 25 gut zu erkennen ist.

Zu erkennen sind insbesondere die den Trägern 30 gegenüberliegenden rückseitigen Arme 28a und 28b, an welchen die Gaszugfedern 29a - 29d befestigt sind.

Weiter ist die drehbare Stange 34 zu erkennen, die als Gegenlager für das untere Ende der Gasfedern 29a - 29d dient.

Es versteht sich, dass statt einer drehbaren Stange alternativ möglich ist, dass jedes untere Ende einer Gaszugfeder 29a - 29d drehbar auf der Stange sitzt.

Für jeden Arm 28a, 28b sind jeweils zwei Gaszugfedern vorgesehen. Die Verwendung mehrerer Gaszugfedern ist insbesondere vorteilhaft, da auch im Falle des Ausfalls einer Gaszugfeder das Winkelteil nicht mit seinem gesamten Gewicht herunterklappen kann, was ansonsten einer Verletzungsgefahr nach sich ziehen könnte.

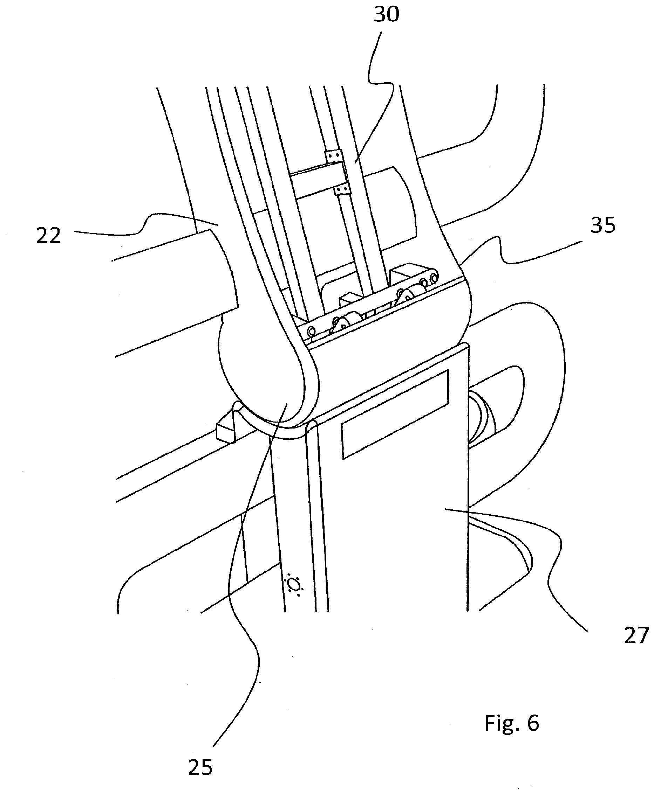

Fig. 6 ist eine Drahtdarstellung des Bereichs des Drehgelenks 25.

Zu erkennen ist das Gehäuseteil 35 des Winkelteils 22, welches sich um die Träger 30 erstreckt. Das Drehgelenk 25 ist in dieser Darstellung in geöffnetem Zustand gezeigt. Das Gehäuseteil 35 ist im Bereich des Drehgelenks 25 kreiszylinderförmig ausgebildet und dreht sich in einer korrespondierenden Ausnehmung des Rückenteils 27.

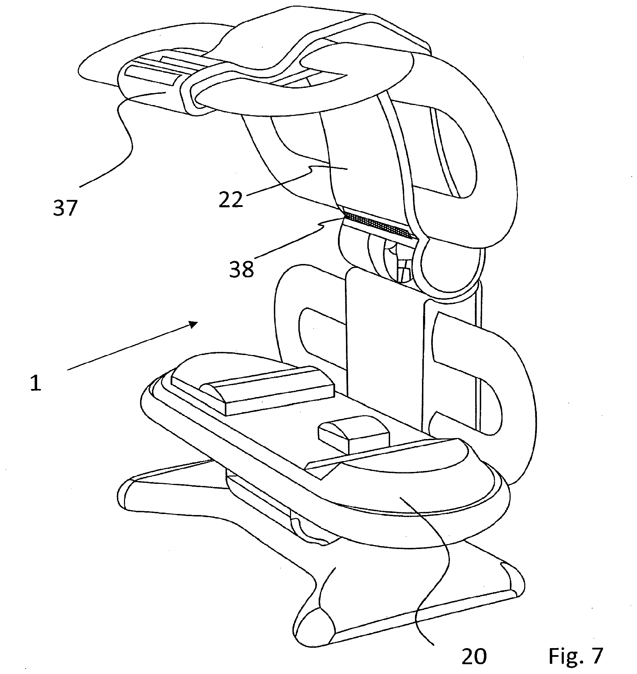

Fig. 7 ist eine perspektivische Ansicht der Vorrichtung zur Kernspinresonanztherapie 1 in geöffnetem Zustand.

Zu erkennen ist, dass das Winkelteil 22, welches zwei Spulen trägt, um nahezu 90° nach oben geklappt ist. So ist ein gut zugängliches Behandlungsvolumen sichergestellt.

Bei einer Ausführungsform der Erfindung ist auch denkbar, dass der Öffnungswinkel durch den Bediener der Vorrichtung 1 einstellbar ist, etwa um die Maximalhöhe des Griffs 37 unterschiedlichen Bedienern anpassen zu können.

Weiter umfasst in diesem Ausführungsbeispiel das Winkelteil 22 sich gegenüberliegende Leuchtstreifen 38. In dieser Ansicht ist nur ein Leuchtstreifen 38 erkennbar. Ein gegenüberliegender Leuchtstreifen ist über der Liegefläche auf der anderen Seite am Winkelteil 22 angeordnet. Die Leuchtstreifen 38 sind vorzugsweise als LED-Beleuchtung ausgebildet, deren Farbe veränderbar ist.

Die Leuchtstreifen 38 können sowohl zur Markierung des Behandlungsvolumens als zur Anzeige dienen, ob die Vorrichtung 1 in Betrieb ist. So kann ich z.B. bei Inbetriebnahme der Vorrichtung 1 die Lichtfarbe ändern.

Fig. 8 zeigt eine perspektivische Ansicht einer weiteren Ausführungsform einer Vorrichtung zur Kernspinresonanztherapie 1.

Die Vorrichtung zur Kernspinresonanztherapie 1 umfasst eine Liege 2, welche in diesem Ausführungsbeispiel horizontal angeordnet ist und auf welche sich ein zu behandelnder Patient (nicht dargestellt) mit seinem gesamten Körper legen kann.

Die Liege 2 ist Teil eines Moduls mit dem Gehäuse 3. Angrenzend an die Liege 2 sowie angrenzend an das Gehäuse 3 sind die Seitenwände 5 und 6 angeordnet. In den Seitenwänden 5 und 6 befinden sich, wie im Folgenden noch im Detail dargestellt wird, die Sweepspulen.

Zu erkennen ist in dieser Darstellung ferner auch das Außengehäuse 7, welches Teil des Gehäuses ist, das die Seitenwand 6 aufnimmt.

Oberhalb der Seitenwände 5, 6 befindet sich ein Spulengehäuse 8 zur Erzeugung eines magnetischen Wechselfeldes.

Eine weitere, hier nicht zu sehende Spule zur Erzeugung eines magnetischen Wechselfeldes befindet sich unterhalb der Liege 2.

Weiter umfasst das Modul mit der Liege ein Steuergerät 4, welches an der Vorderseite dieses Moduls angeordnet ist.

Fig. 9 zeigt eine Drahtdarstellung der in Fig. 8 gezeigten Vorrichtung zur Kernspinresonanztherapie 1, wobei nunmehr auch verdeckte Kanten und damit das Innenleben der

Vorrichtung zur Kernspinresonanztherapie 1 in Art eines Röntgenbildes zu erkennen sind.

Zu erkennen sind nunmehr die beiden Sweepspulen 9 und 10, welche in den Seitenwänden 16, 17 angeordnet sind.

Weiter ist in dieser Darstellung zu erkennen, dass sich die Seitenwand 6 zwischen zwei Säulen 17 befindet und nach unten gefahren werden kann, so dass sich dann die Sweepspule 10 in der Position 10a befindet.

Um das Seitenteil herunterzufahren, sind verschiedene technische Möglichkeiten denkbar. So ist beispielsweise ein manuelles Herunterfahren denkbar. Hierzu kann das Seitenteil in Schienen laufen und über ein Seilzug und eine Umlenkrolle mit einem Gegengewicht verbunden sein. Das Gegengewicht verhindert so ein Herunterrutschen des Seitenteils und ermöglicht ein einfaches Hoch- und Runterfahren per Hand.

Weiter ist auch eine motorische Betätigung denkbar, beispielsweise mittels eines Spindelantriebs.

Die Sweepspulen 9 und 10 sind in Helmholtz-Konfiguration gegenüberliegend angeordnet und nehmen einen Großteil der Länge der Liegefläche ein.

Auch das Spulengehäuse 8 erstreckt sich über einen großen Teil der Länge der Liege und nahezu die gesamte Breite der Liege.

Fig. 10 zeigt die in Fig. 8 und Fig. 9 dargestellte Vorrichtung zur Kernspinresonanztherapie 1, wobei nunmehr das obere Spulengehäuse 8, welches eine Spule zur Erzeugung eines magnetischen Wechselfeldes umfasst, nach oben geklappt ist. Hierzu ist das Spulengehäuse 8 an der Seitenwand 5 angeschlagen.

Wird nunmehr die Seitenwand 6 nach unten gefahren, kann der Benutzer 11 bequem in die Vorrichtung zur Kernspinresonanztherapie einsteigen.

Fig. 10 zeigt eine weitere perspektivische Darstellung einer Vorrichtung zur Kernspinresonanztherapie, bei welcher die Liegefläche des Gehäuses 3, welches als Modul ausgebildet ist, entfernt ist.

Zu erkennen ist nunmehr, dass auch unterhalb der Liegefläche eine Spule 12 zur Erzeugung eines magnetischen Wechselfeldes angeordnet ist. Diese ist mit der oberen Spule zur Erzeugung eines magnetischen Wechselfeldes in Helmholtz-Konfiguration synchronisiert.

Es wird nunmehr ein magnetisches Wechselfeld mit recht homogener Feldstärke im Behandlungsvolumen erzielt, welches senkrecht das Feld der in den Seitenteilen angeordneten Sweepspulen überlagert.

Fig. 12 zeigt eine Ansicht einer Vorrichtung zur Kernspinresonanztherapie 1, bei der nunmehr die Sweepspulen 9 und 10 zu erkennen ist, zwischen denen sich auf der Liege 2 das Behandlungsvolumen befindet.

Fig. 13 zeigt die Ausgestaltung der Sweepspulen.

Diese haben die Form eines Segmentbogenfensters, sind also unten gerade und oben gebogen ausgebildet, wobei zwischen dem Bogen und der Unterseite die Spulen gerade geformt sind.

Denkbar ist aber auch, die Spule als Segmentbogen auszugestalten und/oder die eckigen Bereiche zumindest abzurunden.

Fig. 14 zeigt das Spulengehäuse 8, 15 der oberen und/oder unteren Spule zur Erzeugung eines magnetischen Wechselfeldes.

Das Spulengehäuse ist ringförmig mit im Wesentlichen rechteckiger Geometrie ausgestaltet und passt sich im Wesentlichen der Liegefläche an. In dem mittig offenen Gehäuse befindet sich die Spule 12 zur Erzeugung des magnetischen Wechselfeldes.

Fig. 15 zeigt das Gehäuse 3 des Liegenflächenmoduls. Dieses weist eine Kopfstütze 13 und auf der gegenüberliegenden Seite einen Aufnahmeschacht 14 für das Steuergerät auf (4 in Fig. 8).

Dieses Modul kann um 180° gedreht werden, so dass das Steuergerät sich an der gewünschten Seite befindet.

Fig. 16 zeigt eine Schnittansicht einer Vorrichtung zur Kernspinresonanztherapie 1. Zu erkennen ist nunmehr das unterhalb der Liegefläche angeordnete Spulengehäuse 15 zur

Aufnahme der unteren Spule zur Erzeugung eines magnetischen Wechselfeldes. Weiter zu erkennen ist eine Seitenwand 5, in welche eine Sweepspule angeordnet ist.

Das Behandlungsvolumen geht von der Spule 15 aus über die Liege und befindet sich zwischen den Seitenwänden.

Ergänzend sei angemerkt, dass bei einer alternativen Ausführungsvariante, welche nicht in den Zeichnungen dargestellt ist, die mit Bezugszeichen 6 gekennzeichnete Seitenwand mit dem oberen Spulengehäuse (Bezugszeichen 8) verbunden ist und zusammen mit diesen nach oben geklappt werden kann.

Durch die Erfindung konnte eine Vorrichtung zur Kernspinresonanztherapie bereitgestellt werden, mit der sich große Behandlungsareale erreichen lassen, insbesondere mittels der eine Ganzkörperbehandlung möglich ist und bei welcher der Zugang durch den Patienten vereinfacht ist.

### Bezugszeichenliste

- 1: Vorrichtung zur Kernspinresonanztherapie
- 2: Liegefläche
- 3: Gehäuse
- 4: Steuergerät
- 5: Seitenwand
- 6: Seitenwand
- 7: Außengehäuse
- 8: Spulengehäuse
- 9: Sweepspule
- 10, 10a-b: Sweepspule
- 11: Benutzer
- 12: Spule
- 13: Kopfstütze
- 14: Aufnahmeschacht
- 15: Spulengehäuse
- 16: Säule
- 17: Säule
- 20: Liege
- 21: Fußteil
- 22: Winkelteil
- 23a,23b: Spulengehäuse
- 24: Spulengehäuse
- 25: Drehgelenk
- 26: Bildschirm
- 27: Rückenteil
- 28a,28b: Arm
- 29a-29d: Gaszugfeder
- 30: Träger
- 31: Drehgelenk
- 32: Rahmen
- 33: Vorsprung
- 34: Stange
- 35: Gehäuseteil
- 36: Aussparung
- 37: Griff
- 38: Leuchtstreifen

## Patentansprüche

1. Vorrichtung zur Kernspinresonanztherapie (1), umfassend eine Liege (20) mit einer Liegefläche (2), wobei seitlich neben der Liege Sweepspulen (9, 10) angeordnet sind, wobei durch den Raum zwischen den Spulen (9, 10) ein Behandlungsvolumen definiert wird und wobei unter der Liege (20) eine erste weitere Spule (10a) zur Erzeugung eines magnetischen Wechselfeldes angeordnet ist, wobei über dem Behandlungsvolumen eine zweite weitere Spule (10b) in einem Spulengehäuse (24) zur Erzeugung eines magnetischen Wechselfeldes angeordnet ist, wobei das Behandlungsvolumen ferner durch den Bereich zwischen der Liegefläche (2) bis zum Spulengehäuse (24) definiert ist, wobei senkrecht zu dem durch die Sweepspulen erzeugten Sweepfeld das magnetische Wechselfeld erzeugt wird, und wobei die Feldstärke des gesweepten Feldes zwischen 0,1 bis 100 mT beträgt bei einer Frequenz des Wechselfeldes von 1 bis 100 kHz.

2. Vorrichtung zur Kernspinresonanztherapie (1) nach dem vorstehenden Anspruch, **dadurch gekennzeichnet, dass** die zweite weitere Spule (10b) hochklapp- oder hochfahrbar ausgebildet ist.

3. Vorrichtung zur Kernspinresonanztherapie (1) nach dem vorstehenden Anspruch, **dadurch gekennzeichnet, dass** zumindest die zweite weitere Spule (10b) in einem ringförmigen innen offenen Gehäuse (24) mit vorzugsweise in einer Draufsicht im Wesentlichen rechteckigen Form angeordnet ist.

4. Vorrichtung zur Kernspinresonanztherapie (1) nach einem der vorstehenden Ansprüche, **dadurch gekennzeichnet, dass** zwei Spulen (10, 10b), insbesondere eine Sweepspule und die zweite weitere Spule, oberhalb des Behandlungsvolumens, an einem hochklappbaren Winkelteil (22) angebracht sind.

5. Vorrichtung zur Kernspinresonanztherapie (1) nach dem vorstehenden Anspruch, **dadurch gekennzeichnet, dass** das Winkelteil (22) über ein Drehgelenk (25) mit der übrigen Vorrichtung verbunden ist.

6. Vorrichtung zur Kernspinresonanztherapie (1) nach dem vorstehenden Anspruch, **dadurch gekennzeichnet, dass** das Winkelteil (22) um einen Winkel von mindestens 50°, vorzugsweise von mindestens 70° nach oben schwenkbar ausgebildet ist.

7. Vorrichtung zur Kernspinresonanztherapie nach einem der Ansprüche 4 bis 6, **dadurch gekennzeichnet, dass** das Winkelteil zumindest ein Bedienteil, insbesondere einen Bildschirm, umfasst.

8. Vorrichtung zur Kernspinresonanztherapie (1) nach einem der Ansprüche 4 bis 7, **dadurch gekennzeichnet, dass** das Winkelteil (22) mittels zumindest einer, vorzugsweise mittels mehrerer, Gaszugfeder (29a - 29d) in einer nach oben geklappten Position haltbar ausgebildet ist.

9. Vorrichtung zur Kernspinresonanztherapie (1) nach einem der vorstehenden Ansprüche, **dadurch gekennzeichnet, dass** zumindest eine Spule (9, 10, 10a, 10b) in einer Vergussmasse, insbesondere in einem Harz, eingebettet ist.

10. Vorrichtung zur Kernspinresonanztherapie (1) nach einem der Ansprüche 2 bis 9, **dadurch gekennzeichnet, dass** die Vorrichtung Mittel zur Erfassung aufweist, ob die zweite weitere Spule (10b) hochgeklappt oder hochgefahren ist.

11. Vorrichtung zur Kernspinresonanztherapie (1) nach einem der vorstehenden Ansprüche, **dadurch gekennzeichnet, dass** die Vorrichtung ein Steuergerät aufweist, mit welchem zumindest bei einem Start der Vorrichtung, vorzugsweise zyklisch, die Stromstärke einer der Spulen (9, 10, 10a, 10b) und/oder die Frequenz eines von zumindest einer der Spulen (9, 10, 10a, 10b) erzeugten Wechselfeldes gemessen wird.

12. Vorrichtung zur Kernspinresonanztherapie (1) nach einem der vorstehen Ansprüche, **dadurch gekennzeichnet, dass** das Behandlungsvolumen einen Länge zwischen 1,4 und 2,5 m, eine Breite zwischen 0,4 und 1 m und/oder eine Höhe zwischen 0,4 und 1 m aufweist.

## Claims

1. An apparatus for nuclear magnetic resonance therapy (1), comprising a bed (20) with a bed surface (2), with sweep coils (9, 10) arranged laterally of the bed, the space between the coils (9, 10) defining a treatment volume, and a first further coil (10a) for generating an alternating magnetic field arranged below the bed (20), wherein a second further coil (10b) is arranged in a coil casing (24) above the treatment volume for generating an alternating magnetic field, wherein the treatment volume is furthermore defined by the area between the bed surface (2) and the coil casing (24), wherein the alternating magnetic field is generated perpendicular to the sweep field generated by the sweep coils, and wherein the field strength of the swept field is between 0.1 and 100 mT at a frequency of the alternating field from 1 to 100 kHz.

2. The apparatus for nuclear magnetic resonance therapy (1) according to the preceding claim, **characterised in that** the second further coil (10b) is adapted for being pivoted upwards or for being raised.

3. The apparatus for nuclear magnetic resonance therapy (1) according to the preceding claim, **characterised in that** at least the second further coil (10b) is arranged in a ring-type casing (24) having an inner opening and preferably a substantially rectangular shape when seen in a plan view.

4. The apparatus for nuclear magnetic resonance therapy (1) according to any one of the preceding claims, **characterised in that** two coils (10, 10b), in particular one sweep coil and the second further coil, are mounted above the treatment volume to an angular part (22) that can be pivoted upwards.

5. The apparatus for nuclear magnetic resonance therapy (1) according to the preceding claim, **characterised in that** the angular part (22) is coupled to the rest of the apparatus via a pivot joint (25).

6. The apparatus for nuclear magnetic resonance therapy (1) according to the preceding claim, **characterised in that** the angular part (22) is adapted for being pivoted upwards about an angle of at least 50°, preferably at least 70°.

7. The apparatus for nuclear magnetic resonance therapy according to any one of claims 4 to 6, **characterised in that** the angular part comprises at least one operating unit, in particular a screen.

8. The apparatus for nuclear magnetic resonance therapy (1) according to any one of claims 4 to 7, **characterised in that** the angular part (22) is adapted for being held in an upwardly pivoted position by at least one and preferably a plurality of traction gas springs (29a - 29d).

9. The apparatus for nuclear magnetic resonance therapy (1) according to any one of the preceding claims, **characterised in that** at least one coil (9, 10, 10a, 10b) is embedded in a potting compound, in particular in a resin.

10. The apparatus for nuclear magnetic resonance therapy (1) according to any one of claims 2 to 9, **characterised in that** the apparatus comprises means for detecting whether the second further coil (10b) is in an upwardly pivoted or raised position.

11. The apparatus for nuclear magnetic resonance therapy (1) according to any one of the preceding claims, **characterised in that** the apparatus comprises a control unit which is used to measure the amperage of one of the coils (9, 10, 10a, 10b) and/or the frequency of an alternating field generated by at least one of the coils (9, 10, 10a, 10b), at least upon start-up of the apparatus, preferably cyclically.

12. The apparatus for nuclear magnetic resonance therapy (1) according to any one of the preceding claims, **characterised in that** the treatment volume has a length between 1.4 and 2.5 m, a width between 0.4 and 1 m, and/or a height between 0.4 and 1 m.

## Revendications

1. Dispositif de thérapie par résonance magnétique nucléaire (1), comportant une table (20) dotée d'un plan de couchage (2), des bobines de vobulation (9, 10) étant disposées sur les côtés et de façon contiguë à la table, l'espace entre les bobines (9, 10) constituant un volume de traitement, et une première bobine additionnelle (10a) étant disposée sous la table (20) aux fins de générer un champ magnétique alternatif, une deuxième bobine additionnelle (10b) étant disposée au-dessus du volume de traitement, dans un boîtier de bobine (24), aux fins de générer un champ magnétique alternatif, le volume de traitement étant en outre défini par la région située entre le plan de couchage (2) jusqu'au boîtier de bobine (24), le champ magnétique alternatif étant généré perpendiculairement au champ de vobulation produit par les bobines de vobulation, et l'intensité de champ du champ vobulé étant comprise entre 0,1 et 100 mT, pour une fréquence du champ alternatif qui est comprise entre 1 et 100 kHz.

2. Dispositif de thérapie par résonance magnétique nucléaire (1) selon la revendication précédente, **caractérisé en ce que** la deuxième bobine additionnelle (10b) peut être relevée ou déplacée vers le haut.

3. Dispositif de thérapie par résonance magnétique nucléaire (1) selon la revendication précédente, **caractérisé en ce qu'**au moins la deuxième bobine additionnelle (10b) est disposée dans un boîtier (24) annu-laire, ouvert à l'intérieur, ayant de préférence une forme sensiblement rectangulaire, vue de dessus.

4. Dispositif de thérapie par résonance magnétique nucléaire (1) selon l'une des revendications précédentes, **caractérisé en ce que** deux bobines (10, 10b), en particulier une bobine de vobulation et la deuxième bobine additionnelle, sont disposées au-dessus du volume de traitement, sur un élément formant un angle (22) pouvant être relevé.

5. Dispositif de thérapie par résonance magnétique nucléaire (1) selon la revendication précédente, **caractérisé en ce que** l'élément formant un angle (22) est relié par une articulation tournante (25) au reste du dispositif.

6. Dispositif de thérapie par résonance magnétique nucléaire (1) selon la revendication précédente, **caractérisé en ce que** l'élément formant un angle (22) est réalisé de manière à pouvoir pivoter vers le haut sous un angle d'au moins 50°, de préférence d'au moins 70°.

7. Dispositif de thérapie par résonance magnétique nucléaire selon l'une des revendications 4 à 6, **caractérisé en ce que** l'élément formant un angle comporte au moins un élément de commande, en particulier un écran.

8. Dispositif de thérapie par résonance magnétique nucléaire (1) selon l'une des revendications 4 à 7, **caractérisé en ce que** l'élément formant un angle (22) est réalisé de manière à pouvoir être maintenu dans une position relevée à l'aide d'au moins un, de préférence à l'aide de plusieurs ressorts de traction à gaz (29a à 29d).

9. Dispositif de thérapie par résonance magnétique nucléaire (1) selon l'une des revendications précédentes, **caractérisé en ce qu'**au moins une bobine (9, 10, 10a, 10b) est noyée dans une masse de scellement, en particulier dans une résine.

10. Dispositif de thérapie par résonance magnétique nucléaire (1) selon l'une des revendications 2 à 9, **caractérisé en ce que** le dispositif comprend des moyens de détection pour déterminer si la deuxième bobine additionnelle (10b) est relevée ou déplacée vers le haut.

11. Dispositif de thérapie par résonance magnétique nucléaire (1) selon l'une des revendications précédentes, **caractérisé en ce que** le dispositif présente un appareil de contrôle avec lequel on mesure, au moins lors d'un démarrage du dispositif, de préférence de manière cyclique, l'intensité de courant de l'une des bobines (9, 10, 10a, 10b) et/ou la fréquence d'un champ alternatif généré par au moins une des bobines (9, 10, 10a, 10b).

12. Dispositif de thérapie par résonance magnétique nucléaire (1) selon l'une des revendications précédentes, **caractérisé en ce que** le volume de traitement présente une longueur comprise entre 1,4 et 2,5 m, une largeur comprise entre 0,4 et 1 m et/ou une hauteur comprise entre 0,4 et 1 m.
